# EUROPÄISCHE PATENTANMELDUNG

(11) **EP 0 653 424 A1**
(43) Veröffentlichungstag der Anmeldung: **17.05.1995**
(21) Anmeldenummer: 94117183.7
(22) Anmeldetag: 31.10.1994
(51) Int. Cl.: C07D 241/38, C01B 31/02

(54) **Amino-ureido- und -thiouredo-fulleren-Derivate und Verfahren zu deren Herstellung**

(30) Priorität: 12.11.1993 DE 4338672
(71) Anmelder: HOECHST AKTIENGESELLSCHAFT, D-65929 Frankfurt am Main (DE)
(72) Erfinder: Kampe, Klaus-Dieter, Dr., D-65812 Bad Soden (DE)

(57) **Zusammenfassung**

Amino-ureido-und Amino-thioureido-Verbindungen der Fullerene C₆₀ und/oder C₇₀ sowie Verfahren zu deren Herstellung und deren Verwendung werden beschrieben.

## Beschreibung

Die Erfindung betrifft neue Amino-ureido- und Amino-thioureido-Verbindungen der Fullerene C₆₀ und/oder C₇₀ sowie Verfahren zu deren Herstellung und deren Verwendung.

Chemische Reaktionen mit den Fullerenen C₆₀ und/oder C₇₀ sind prinzipiell möglich, verlaufen aber, da es sich bei C₆₀ und C₇₀ um polyfunktionelle Moleküle handelt, häufig unter Bildung komplexer, nicht trennbarer Mischungen von Reaktionsprodukten. Das betrifft sowohl die Anzahl der an C₆₀ und/oder C₇₀ eingetretenen Umsetzungen bzw. eingeführten Reste, wie auch die unterschiedlichen relativen Strukturen von möglichen Regioisomeren [A. Hirsch, A. Soi., H.R. Karfunkel, Angew. Chemie 1992, 104, 808; F. Wudl, A. Hirsch, K.C. Khemani, T. Suzuki, P.-M. Allemand, A. Koch, H. Eckert, G. Srdanov, H. Webb, in Fullerenes: Synthesis, Properties and Chemistry of Large Carbon Clusters; Hammond, G. S.; Kuck, V.S., Eds.; Washington, DC, 1992; p. 161.

In der Deutschen Patentanmeldung P 43 12 632.4 werden gut zugängliche, definierte, in reiner Form isolierbare Diamino-Derivate der Fullerene C₆₀ und C₇₀ beschrieben, die neben einer tertiären Aminogruppe eine NH-Funktion enthalten.

Folgereaktionen mit Fulleren-Derivaten werden sehr häufig erschwert oder versagen ganz wegen der Schwer- oder Unlöslichkeit der Ausgangs-Fulleren-Derivate in den dafür verwendbaren inerten Lösungsmitteln. Auch verlaufen Reaktionen mit C₆₀ und/oder C₇₀-Derivaten oft sehr träge und benötigen höhere Reaktionstemperaturen und lange Reaktionszeiten, die häufig wegen der unzureichenden Stabilität der Fulleren-Edukte und/oder der herzustellenden Fulleren-Produkte nicht anwendbar sind.

Aufgrund der oftmals nur mit sehr aufwendiger Arbeitsweise zugänglichen, definierten und charakterisierten synthetischen Fullerenverbindungen sind gezielte Folgereaktionen an funktionellen Fulleren-Derivaten bisher nur in wenigen Fällen erfolgreich ausgeführt worden.

Die Aufgabe besteht darin, gut zugängliche, definierte, in reiner Form isolierbare Amino-ureido- und Amino-thioureido-Verbindungen bereitzustellen.

Gegenstand der Erfindung sind Amino-ureido- oder -thioureido-C₆₀- und/oder C₇₀-Verbindungen der Formel I · 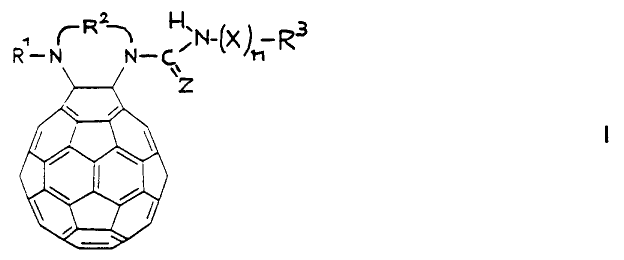
und/oder II, 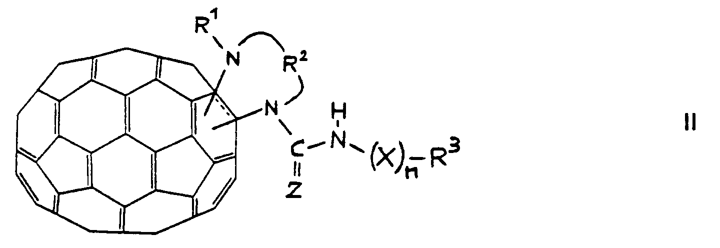
in denen
- R¹: (C₁-C₄)-Alkyl ist,
- R²: (C₂-C₄)-Alkylen oder 1,2-Cyclo-(C₃-C₇)-alkylen ist,
- n: eine ganze Zahl Null oder 1 ist,
- Z: O oder S ist,
- X: (C₁-C₁₂)-Alkylen, (C₃-C₁₂)-Alkenylen oder (C₆-C₁₀)-Arylen ist, wobei Alkylen und Alkenylen gegebenenfalls durch (C₁-C₄)-Alkoxy, (C₁-C₄)-Alkylthio, Cl, Br, J, (C₁-C₄)-Alkoxycarbonyl, (C₆-C₁₀)-Aryl und/oder (C₆-C₁₀)-Aryl-(C₁-C₄)-alkyl substituiert ist,
wobei Aryl gegebenenfalls ein- oder mehrfach unabhängig voneinander substituiert ist durch Halogen, (C₁-C₄)-Alkyl, (C₁-C₄)-Alkoxy, CF₃, CN, oder NO₂,
und wobei Arylen gegebenenfalls ein- oder mehrfach unabhängig voneinander substituiert ist durch Halogen, (C₁-C₄)-Alkyl, (C₁-C₄)-Alkoxy, (C₁-C₄)-Alkylthio, CF₃, OCF₃, CN, NO₂ und/oder (C₁-C₄)-Alkoxycarbonyl,
- R³: falls n Null ist,
(C₁-C₁₂)-Alkyl, (C₃-C₁₁)-Alkenyl, (C₃-C₈)-Alkinyl, (C₁-C₈)-Perfluoralkyl, (C₆-C₁₄)-Aryl oder Trimethylsilyl ist, wobei Alkyl, Alkenyl und Alkinyl gegebenenfalls ein- oder mehrfach unabhängig voneinander durch (C₁-C₄)-Alkoxy, (C₁-C₄)-Alkylthio, Phenyloxy, Phenylthio, Halogen, ClSO₂ und/oder (C₁-C₄)-Alkoxycarbonyl, und Aryl gegebenenfalls ein- oder mehrfach unabhängig voneinander durch Halogen, (C₁-C₄)-Alkyl, welches gegebenenfalls durch Halogen substituiert ist, (C₁-C₄)-Alkoxy, NO₂, CN, OCF₃, ClSO₂, SO₃H, (C₁-C₄)-Alkanoyloxy, (C₁-C₄)-Alkoxycarbonyl und/oder
(C₁-C₂)-Alkylendioxy substituiert ist,
- R³: falls n = 1 ist,
Wasserstoff, CH₃, welches gegebenenfalls durch Halogen substituiert ist, CH = CH₂, (C₁-C₄)-Alkoxy, (C₁-C₄)-Alkylthio, Halogen, (C₆-C₁₄)-Aryl, (C₆-C₁₄)-Aryloxy, (C₆-C₁₄)-Aryl-(C₁-C₄)-alkyloxy, Trimethylsilyloxy, C(O)NHR', C(O)NR'₂, COOR', worin R' (C₁-C₄)-Alkyl bedeutet, (C₁-C₆)-Alkanoyloxy, SO₂R⁵, OSO₂R⁵, worin R⁵ (C₁-C₄)-Alkyl, (C₆-C₁₀)-Aryl oder (C₁-C₄)-Alkyl-(C₆-C₁₀)-aryl bedeutet, oder -N = C = Z, worin Z die obengenannte Bedeutung hat, und Aryl gegebenenfalls ein- oder mehrfach unabhängig voneinander durch Halogen, (C₁-C₄)-Alkoxy, (C₁-C₄)-Alkylthio, (C₁-C₄)-Alkyl, welches gegebenenfalls durch Halogen substituiert ist, NO₂, CN, ClSO₂, SO₃H, (C₁-C₄)-Alkanoyloxy, (C₁-C₄)-Alkoxycarbonyl oder (C₆-C₁₀)-Aryloxy substituiert ist.

### Bevorzugte Verbindungen der Formel I und II sind solche, in denen

- Z: 0 ist,
- X: (C₁-C₈)-Alkylen, gegebenenfalls durch (C₁-C₄)-Alkoxy, (C₁-C₄)-Alkylthio, (C₁-C₄)-Alkoxycarbonyl oder Cl substituiert, oder o-, m- oder p-Phenylen ist, gegebenenfalls substituiert durch Halogen, (C₁-C₄)-Alkoxy, (C₁-C₄)-Alkyl oder CF₃, und
- R³: falls n Null ist,
(C₁-C₁₂)-Alkyl, Allyl, Phenyl, Benzyl oder Naphthyl ist, wobei Alkyl gegebenenfalls unabhängig voneinander einfach oder zweifach durch (C₁-C₄)-Alkoxy, (C₁-C₄)-Alkylthio, Phenyloxy, Phenylthio, Cl, Br oder (C₁-C₄)-Alkyloxycarbonyl substituiert ist, und Phenyl, Benzyl oder Naphthyl gegebenenfalls ein- oder mehrfach unabhängig voneinander durch Halogen, (C₁-C₄)-Alkoxy, (C₁-C₄)-Alkyl, CF₃, NO₂, CN, (C₁-C₄)-Alkanoyloxy, ClSO₂, SO₃H und/oder (C₁-C₄)-Alkoxycarbonyl oder einfach durch Methylendioxy substituiert ist, und
- R³: falls n = 1 ist,
Wasserstoff, CH₃, ClCH₂, BrCH₂, Vinyl, (C₁-C₄)-Alkoxy, (C₁-C₄)-Alkylthio, Cl, Br, Phenoxy, Benzyloxy, Phenyl, Naphthyl, C(O)NHR', C(O)NR'₂ oder COOR', worin R' (C₁-C₄)-Alkyl bedeutet, ist und wobei Phenyl oder Naphthyl gegebenenfalls ein- oder mehrfach unabhängig voneinander durch (C₁-C₄)-Alkoxy, (C₁-C₄)-Alkyl, CF₃, NO₂, CN, ClSO₂, SO₃H und/oder (C₁-C₄)-Alkoxycarbonyl substituiert ist, und R¹, R² und n die obengenannte Bedeutung haben.

### Besonders bevorzugt sind Verbindungen der Formel I und II, in denen

- R¹: CH₃ oder C₂H₅ ist,
- R²: (C₂-C₃)-Alkylen oder 1,2-Cyclo-(C₅-C₆)-alkylen ist,
- n: eine ganze Zahl Null oder 1 ist,
- Z: O ist,
- X: (C₁-C₇)-Alkylen, gegebenenfalls durch CH₃O, CH₃S, COOCH₃ oder COOC₂H₅ substituiert, oder o-, m- oder p-Phenylen ist,
- R³: falls n Null ist,
(C₁-C₈)-Alkyl, Allyl, Phenyl, Benzyl oder Naphthyl ist, wobei Alkyl gegebenenfalls einfach oder zweifach unabhängig voneinander durch CH₃O, CH₃S, COOCH₃ oder COOC₂H₅ substituiert ist, und Phenyl, Benzyl oder Naphthyl gegebenenfalls einfach oder zweifach unabhängig voneinander durch F, Cl, Br, CH₃O, C₂H₅O, CH₃, C₂H₅, NO₂, ClSO₂, SO₃H, CF₃, COOCH₃ und/oder COOC₂H₅ oder 1,2-O-CH₂-O substituiert ist, und
- R³: falls n = 1 ist,
Wasserstoff, CH₃, CH₃O, CH₃S, Cl, Br, C₆H₅O, C₆H₅CH₂O, Phenyl, COOCH₃, COOC₂H₅, C(O)NHR', C(O)N-R'₂, wobei R' (C₁-C₄)-Alkyl bedeutet, ist, und Phenyl gegebenenfalls durch CH₃O, C₂H₅O, CH₃, CF₃, NO₂, ClSO₂, COOCH₃ oder COOC₂H₅ substituiert ist.

Ganz besonders bevorzugt sind Verbindungen der Formel I, worin R¹, R², n, Z, X und R³ die vorstehend genannte Bedeutung haben.

Kohlenwasserstoffreste wie Alkyl, Alkylen, Alkenyl, Alkenylen, Alkinyl oder auch Alkoxy können verzweigt oder unverzweigt sein. Aryl bedeutet beispielsweise Phenyl oder Naphthyl. Arylalkyl bedeutet beispielsweise Benzyl. Arylen bedeutet z.B. Phenylen. Aryl kann ein- oder mehrfach bevorzugt ein-, zwei-, drei- oder vierfach, besonders bevorzugt ein- oder zweifach, substituiert sein. In diesem Zusammenhang ist unter einem substituierten Arylrest beispielsweise auch ein substituierter Aryloxyrest zu verstehen. Unter Perfluoralkyl ist ein vollständig fluorierter Alkylrest zu verstehen. Halogen bedeutet Fluor, Chlor, Brom oder Jod. (C₁-C₂)-Alkylendioxy als Substituent von Phenyl bedeutet einen 1,2-O-(CH₂)ₘ-O-Rest, wobei m 1 oder 2 ist.

Die Erfindung betrifft ferner ein Verfahren zur Herstellung der Fulleren-Verbindungen der Formeln I und/oder II, 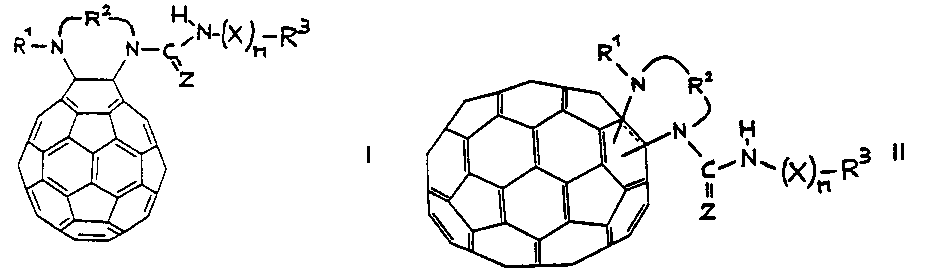
worin R¹, R², n, Z, X und R³ die obengenannte Bedeutung haben, das dadurch gekennzeichnet ist, daß man Verbindungen der Formeln III und/oder IV 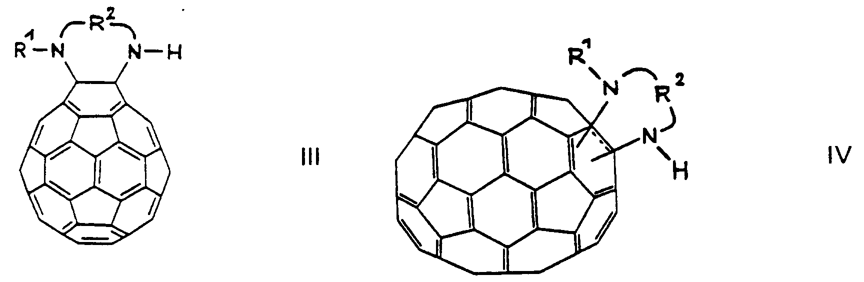
in denen
R¹ und R² die obengenannte Bedeutung haben, mit einem Isocyanat oder Isothiocyanat der Formel V

Z = C = N - (X)ₙ - R³ V

in der Z, n, X und R³ die obengenannte Bedeutung haben, zu Verbindungen der Formel I und/oder II umsetzt.

Besonders bevorzugt ist die Herstellung von Verbindungen der Formel I, aus Verbindungen der Formel III und V.

Die erfindungsgemäße Umsetzung wird bevorzugt in einem gegenüber den Verbindungen der Formeln I, II, III, IV und V inerten, vorzugsweise aromatischen, Löse- oder Suspensionsmittel, bzw. einem Gemisch solcher Löse- oder Suspensionsmittel, ausgeführt.

Als Löse- oder Suspensionsmittel sind beispielsweise geeignet: Toluol, Benzol, Xylole, Anisol, Chlorbenzol, Dichlorbenzole, Methylnaphthaline, Chlornaphthaline, Brombenzol, Tetralin, Dimethylnaphthaline, Methylthiophene und/oder Tetrachlorethane.

Die erfindungsgemäße Umsetzung kann bei einer Temperatur von -30°C bis 300°C, vorzugsweise + 30°C bis 115°C, durchgeführt werden. Die Umsetzung kann sowohl in Lösung als auch in Suspension erfolgen, d.h. die Ausgangsstoffe, die Reaktanden, sowie die erfindungsgemäßen Verbindungen der Formel I und/oder II können in der Reaktionsmischung in gelöster oder ungelöster Form vorliegen. Die Umsetzung kann aber auch bei einer Temperatur tiefer als -30°C bzw. höher als +300°C stattfinden.

Das Molverhältnis zwischen den Fulleren-Derivaten der Formel III und/oder IV und den Isocyanaten oder Isothiocyanaten der Formel V kann bei dem erfindungsgemäßen Verfahren 1:1 bis 1:1000, bevorzugt 1:3 bis 1:200, besonders bevorzugt 1:6 bis 1:30, betragen, oder auch noch kleiner 1:1000 sein.

Die Umsetzung der Fulleren-Derivate der Formeln III und/oder IV mit den Isocyanaten der Formel V (Z = O) verläuft überraschend schnell und selektiv unter Entstehung der Verbindungen der Formel I bzw. II, selbst in heterogenen Systemen.

Die Umsetzung von Verbindungen der Formel III und/oder IV mit Isothiocyanaten der Formel V, worin Z = S ist, verläuft im Vergleich zur Reaktion mit den Isocyanaten der Formel V, worin Z = O ist, mit geringerer Reaktionsgeschwindigkeit.

Das Molverhältnis zwischen den Isocyanaten oder Isothiocyanaten der Formel V und den Fulleren-Derivaten der Formel III und/oder IV, die Reaktionstemperatur und die Reaktionsgeschwindigkeit bei dem erfindungsgemäßen Verfahren stehen in dem bekannten Zusammenhang:
a) je größer das Molverhältnis, desto niedriger kann die Reaktionstemperatur gehalten bzw. gewählt werden, bzw.
b) je größer das Molverhältnis und je höher die Reaktionstemperatur umso größer die Reaktionsgeschwindigkeit, was kürzere Reaktionszeiten erlaubt. Dieser letzte Gesichtspunkt ist speziell bei der Umsetzung der reaktionsträgeren Isothiocyanate der Formel V (Z = S) von Interesse.

Die Reaktionszeiten können gemäß vorstehend angeführter Parameter innerhalb eines breiten Bereichs variieren, sie liegen in der Regel zwischen 10 Minuten und 5 Tagen, je nachdem, ob Isocyanate der Formel V (Z = O) oder Isothiocyanate der Formel V (Z = S) zur Reaktion kommen. Es können aber auch kürzere oder längere Reaktionszeiten ausreichend bzw. nötig sein.

Die erfindungsgemäßen, Amino-ureido- bzw. -thioureido-Fulleren-Derivate der Formel I und II sind durch ihre chemischen Eigenschaften, chemische Zusammensetzung und ihre spektroskopischen Daten einwandfrei charakterisiert.

Unter den chemischen Eigenschaften dient das Verhalten bei Chromatographien, wie konventioneller Dünnschicht- und Säulenchromatographie sowie HPLC zur Charakterisierung der gebildeten neuen Verbindungen.

Die chemische Zusammensetzung der erfindungsgemäßen Fulleren-Derivate der Formel I und II geht aus den Elementaranalysen hervor.

Weiterhin dienen im besonderen Maß die spektroskopischen Daten der neuen erfindungsgemäßen Verbindungen zu deren eindeutiger Charakterisierung. Dazu zählen die Absorptionsspektren im UV-, sichtbaren- und IR-Bereich. Die erfindungsgemäßen Fulleren-Derivate zeigen charakteristische IR-Spektren mit scharfer Bandenstruktur. Ebenso haben die erfindungsgemäßen Verbindungen jeweils charakteristische UV-Absorptionen, unterscheiden sich also durch die Lage ihrer Maxima.

Ebenso charakterisieren die Massenspektren, aufgenommen in der FAB (fast atom bombardment) MS-Technik die jeweiligen Verbindungen und bestätigen soweit vorhanden bzw. erkennbar durch den Molpeak die Molekulargewichte.

Auch die NMR-Spektren, gemessen sowohl als Festkörper- wie auch insbesondere als Lösungsspektren, dienen zur Charakterisierung und Strukturzuordnung der erfindungsgemäßen Verbindungen.

Zum Zweck der Isolierung und Reinigung können die erfindungsgemäßen Fulleren-Derivate der Formel I und/oder II durch Einengen der Reaktionslösung partiell als kristalline Stoffe abgeschieden und als solche in üblicher Weise, z.B. durch Filtration, isoliert werden.

Eine bevorzugte Ausführungsform zur Isolierung und Reinigung der erfindungsgemäßen Verbindungen der Formel I und/oder II besteht darin, daß die Reaktionsmischung entweder direkt oder nach vorheriger Filtration durch Säulenchromatographie, vorzugsweise an Kieselgel, in gegebenenfalls aus den Verbindungen der Formel V entstandene Nebenprodukte, nichtumgesetztes Fulleren-Derivat der Formel III und/oder IV und die gebildeten Amino-ureido- bzw. thioureido-Fullerene der Formel I und/oder II aufgetrennt wird. Vorteilhaft wird die Säulenchromatrographie an Kieselgel mit Toluol und Dichlormethan und Toluol/Methanol oder Dichlormethan/Methanol-Mischungen als Fließmittel ausgeführt.

Überwiegend laufen bei Dünnschichtchromatographien (DC) auf Kieselgel die erfindungsgemäßen Verbindungen der Formel I in den Fließmitteln CH₂Cl₂/C₂H₅OH (Volumenverhältnis: 100/4) und Toluol/Methanol (Volumenverhältnis: 100/3) langsamer - haben also kleinere R_{F}-Werte - als die Ausgangs-Fulleren-Derivate der Formel III. Auch bei der säulenchromatographischen Reinigung an Kieselgel 60 (Korngröße 0,040-0,063 mm) laufen, sofern noch vorhanden, unumgesetzte Ausgangs-Fulleren-Derivate der Formel III in der Regel vor den erfindungsgemäßen Fulleren-Derivaten der Formel I.

Solche chromatographische Trennungen sind andererseits auch nach der "Reversedphase (RP)"-Technik mit einem entsprechenden Kieselgel oder mit anderen Adsorptionsmitteln als stationärer Phase z.B. Al₂O₃ möglich. Nach Abdampfen des Elutionsmittels fallen die erfindungsgemäßen Additionsverbindungen als feste, häufig kristalline Stoffe an. Sofern erforderlich können in diesem Stadium gegebenenfalls noch vorhandene Nebenprodukte, die sich aus den verwendeten Isocyanaten oder Isothiocyanaten der Formel V, beispielsweise durch Feuchtigkeitseinwirkung gebildet haben, durch Digerieren mit aprotischen polaren Lösemitteln, wie z.B. Ether, Tetrahydrofuran, Acetonitril und/oder Aceton, entfernt werden. In solchen Lösemitteln sind die gebildeten erfindungsgemäßen Fulleren-Derivate der Formel I und/oder II im allgemeinen schwer oder quasi unlöslich.

Es ist auch ein Merkmal dieser Erfindung, daß sie einen einfachen Bildungsweg zu den Amino-ureido-bzw.- thioureido-fulleren-Derivaten der Formel I und II zum Gegenstand hat und, daß die Reaktionsprodukte der Formel I und/oder II, insbesondere die Verbindungen der Formel I, auf einfache und kostengünstige Art und Weise, durch konventionelle Säulenchromatographie, also ohne Einsatz von apparativ aufwendiger und nur zur Herstellung kleiner Mengen geeigneter Hochdruckflüssigkeitschromatographie, in reiner Form isolierbar sind. Die durch Säulenchromatographie oder durch andere Aufarbeitungsmethoden erhaltenen erfindungsgemäßen Verbindungen können im Bedarfsfall durch Umkristallisation weiter gereinigt werden.

Obgleich man im Rahmen der vorliegenden Erfindung bei der Auftrennung, Isolierung und Reinigung der erfindungsgemäßen Verbindungen überraschenderweise nicht auf den Einsatz von HPLC-Technik angewiesen ist, ist die HPLC-Technik zur Charakterisierung der erfindungsgemäß erhaltenen Additionsverbindungen geeignet. Retentionszeit gekoppelt mit der verwendeten stationären und der flüssigen Phase, der Flußgeschwindigkeit sowie den üblichen Säulenparametern dienen als verläßliche Stoffparameter zur Charakterisierung von erfindungsgemäßen Reinsubstanzen oder auch Gemischen.

Ein weiterer Vorteil der erfindungsgemäß möglichen Aufarbeitung der Reaktionsmischung durch Säulenchromatographie besteht darin, daß gegebenenfalls nicht-umgesetzte Ausgangs-Fulleren-Derivate einfach und sauber abgetrennt und damit zur Wiederverwendung zurückgewonnen werden können. Das ist in Anbetracht des hohen Preises von Fullerenverbindungen von erheblicher Bedeutung.

Die als Ausgangstoffe erforderlichen Fulleren-Derivate der Formel III und/oder IV können gemäß dem in der Deutschen Patentanmeldung P 43 12 632.4 und dem bei Kampe et al. [Angew. Chem. Int. Ed. (Engl.) 32, 1174 (1993)] beschriebenen Verfahren hergestellt werden. Die für die Umsetzung erforderlichen Isocyanate und Isothiocyanate der Formel V sind bekannt oder können nach bekannten Methoden hergestellt werden.

Die erfindungsgemäßen Fulleren-Derivate eignen sich zur Verwendung als Komplexliganden. In dieser Eigenschaft sind sie zur Modifizierung von Katalysatoren verwendbar.

Weiterhin können die erfindungsgemäßen Verbindungen als solche oder in modifizierter Form zur Hemmung von Enzymen, beispielsweise zur Hemmung von HIV (human immunodeficiency virus)-Enzymen, wie HIV-1-Protease, Verwendung finden, womit sie potentielle biologische Wirkstoffe darstellen, die beispielsweise als antivirale Mittel einsetzbar sind.

Die folgenden Beispiele dienen zur Erläuterung der Erfindung, ohne diese auf die beispielhaft genannten Bedingungen einzuschränken.

Sofern in den folgenden Beispielen nicht anders beschrieben, wurden die Säulenchromatographien an Kieselgel 60, Korngröße 0,040 bis 0,063 mm, der Firma E. Merck, Darmstadt und die Dünnschichtchromatographie (DC) auf Kieselgel 60 F₂₅₄ (Schichtdicke 0,25 mm) der Firma Riedel-de Haen AG, Seelze ausgeführt.

### Beispiel 1

Eine Lösung von 180 mg (0,227 mmol) einer Fullerenverbindung der Formel III, in der R¹ CH₃ und R²-CH₂CH₂- bedeutet, in 450 ml Toluol wurde mit 0,25 ml (242 mg; 4,24 mmol) Methylisocyanat versetzt und 2,1 Tage bei 37-40°C und 2,7 Tage bei RT gerührt. Danach engte man die klare Reaktionslösung im Vakuum bei 40°C auf ca. 40 % des ursprünglichen Volumens ein und ließ diese Lösung auf eine Kieselgel 60/Toluol-Säule (H:42 cm; Ø 2,0 cm) aufziehen. Nach Elution mit 50 ml Toluol, wobei Spuren C₆₀ (violette Farbe) von der Säule liefen, wurde mit CH₂Cl₂ (200 ml), CH₂Cl₂/CH₃OH (100:1) (600 ml); (100:2) (300 ml) eluiert, wobei nach Abdampfen des Elutionsmittels 140 mg Eluatrückstand anfielen, der in Toluol gelöst über Clarcel (Filtrierhilfe) filtriert wurde. Nach dem Einengen im Vakuum wurde der verbleibende Rückstand in Ether suspendiert, abgesaugt, mit Ether nachgewaschen und 8 Stunden bei 80°C, 6-8 mbar getrocknet. Man erhielt 124 mg (= 64,3 % Ausbeute) einer Verbindung der Formel I, in der R¹ und R³: CH₃; R²: -CH₂CH₂-; n = Null und Z: O bedeuten.

| C₆₅H₁₁N₃O (849,83) | | | | |
|---|---|---|---|---|
| berechnet: | C 91,87 | H 1,30 | N 4,94 | % |
| gefunden: | C 88,9 | H 1,4 | N 4,5 | % |

IR-Spektrum (Pulver auf KBr) : *v*_{C=O}1703 cm⁻¹ (intensivste IR-Bande)
Das Massenspektrum (FAB) zeigt eine Molmasse M = 849 (intensiver M^{⊖}-Peak bei m/z 849)
Dünnschichtchromatographie: Fließmittel CH₂Cl₂/C₂H₅OH: 100/4
R_{F}: 0,25 bis 0,30.

### Beispiel 1a

Die gleiche Umsetzung wie vorstehend beschrieben wurde anstelle in Toluol in 500 ml Benzol durchgeführt, wobei die Reaktionszeit 3,5 Tage bei 38-41°C und 3,1 Tage bei RT betrug. Nach gleicher Aufarbeitung wie vorstehend beschrieben wurden nach dem Trocknen 102 mg (52,9 % Ausb.) dieser Fullerenverbindung der Formel I erhalten.

### Beispiel 2

Eine Suspension von 100 mg (0,124 mmol) einer Fullerenverbindung der Formel III, in der R¹ C₂H₅ und R² -CH₂CH₂- bedeutet, in 100 ml Toluol wurde bei 70°C mit einer Lösung von 357 mg (2,3 mmol) Octylisocyanat in 5 ml Toluol versetzt und 0,82 Tage bei 70-72°C und 1 Tag (24 Stunden) bei RT gerührt. Danach wurde die Reaktionslösung im Vakuum eingedampft. Den Rückstand suspendierte man in Ether und saugte ab. Der Filterrückstand (Feststoff 103 mg) wurde in 20 ml Toluol gelöst. Diese Lösung ließ man auf eine Kieselgel 60/Toluol-Säule (H: 45; Ø 2,4 cm) aufziehen. Die Chromatographie wurde bei 0,35 bar Stickstoff-Überdruck durchgeführt. Nach Elution mit je 500 ml Toluol; Toluol/Methanol (100:0,1) und (100:0,2) wurden durch weitere Elution mit 700 ml Toluol/Methanol (100:0,2) nach dem Eindampfen 77 mg Eluatrückstand erhalten. Dieser wurde in Ether suspendiert, abgesaugt und 8 Stunden bei 80°C, 4-6 mbar getrocknet. Man erhielt so 68 mg (= 57 % Ausbeute) einer Verbindung der Formel I, in der R¹: C₂H₅; R²: -CH₂CH₂-; n = Null, Z: O und R³: n-C₈H₁₇ bedeuten.

| C₇₃H₂₇N₃O (962,04) | | | | |
|---|---|---|---|---|
| berechnet: | C 91,14 | H 2,83 | N 4,37 | % |
| gefunden: | C 89,9 | H 3,3 | N 4,3 | % |

Das Massenspektrum (FAB) zeigt eine Molmasse M = 961 (intensiver M^{⊖}-Peak bei m/z 961)
DC: Fließmittel Toluol/Methanol: 100/3: R_{F}: 0,45-0,49.

### Beispiel 3

Eine Suspension von 317 mg (0,4 mmol) einer Fullerenverbindung der Formel III, in der R¹ CH₃ und R² -CH₂CH₂- bedeutet, in 250 ml Toluol wurde bei 70°C mit einer Lösung von 1,0 g (8,4 mmol) Phenylisocyanat in 10 ml Toluol versetzt und 4,7 Stunden. bei 70°C gerührt. Danach wurde die Reaktionslösung im Vakuum eingedampft, der verbleibende Rückstand in Ether suspendiert, der Feststoff abgesaugt, mit Ether ausgewaschen und im Vakuum getrocknet. Man erhielt 428 mg Feststoff, der in 50 ml Toluol gelöst auf eine Kieselgel 60/Toluol-Säule (H44; Ø 2,0 cm) aufgezogen wurde. Die Elution erfolgte bei 0,35 bar N₂-Überdruck mit 200 ml Toluol; 500 ml CH₂Cl₂; 500 ml CH₂Cl₂/CH₃OH (100:1) und 200 ml CH₂Cl₂/CH₃OH (100:1,5). Bei weiterer Elution mit 300 ml CH₂Cl₂/CH₃OH (100:1,5) und 700 ml CH₂Cl₂/CH₃OH (100:2) fielen nach Verdampfen der Fließmittel 365 mg Eluatrückstand an, der in Ether suspendiert und abgesaugt wurde. Nach dem Trocknen (8 Stunden, 80°C, 5-7 mbar) erhielt man 351 mg (96,2 % Ausbeute) einer Verbindung der Formel I, in der R¹: CH₃; R²: -CH₂CH₂-; n = Null, Z: O und R³:C₆H₅ bedeuten.

| C₇₀H₁₃N₃O (911,90) | | | | |
|---|---|---|---|---|
| berechnet: | C 92,20 | H 1,44 | N 4,61 | % |
| gefunden: | C 90,1 | H 1,5 | N 4,2 | % |

Das Massenspektrum (FAB) zeigt eine Molmasse M = 911 (intensiver M^{⊖}-Peak bei m/z 911)
DC: Toluol/Methanol: 10/1: R_{F}: 0,39-0,42
IR-Spektrum: *v*_{C=O}1706; *v*_{NH,C-N}1414 cm⁻¹

### Beispiel 4

Eine Suspension von 160 mg (0,2 mmol) einer Verbindung der Formel III, in der R¹ CH₃ und R² -CH₂CH₂- bedeutet, in 150 ml Toluol wurde bei 70°C mit einer Lösung von 600 mg (4,44 mmol) Phenylisothiocyanat in 8 ml Toluol versetzt und 10,1 Tage bei 65-68°C und 5,9 Tage bei RT gerührt. Danach wurde im Vakuum eingedampft, der verbleibende Rückstand in Ether suspendiert, der Feststoff abgesaugt und in 75 ml CS₂ gelöst. Diese Lösung wurde auf eine Kieselgel 60/Toluol-Säule (H: 40; Ø 2,2 cm) aufgezogen. Die Elution erfolgte bei 0,35 bar N₂-Überdruck mit 500 ml Toluol und Toluol/Methanol (500 ml; 100:0,1 und 1l, 100:0,2). Bei weiterer Elution mit 500 ml Toluol/Methanol (100:0,2) fielen nach Verdampfen des Fließmittels 116 mg Eluatrückstand (DC einheitlich) an, der in Ether suspendiert und abgesaugt wurde. Nach dem Trocknen (vgl. Beispiel 3) erhielt man 106 mg (57,1 % Ausbeute) DC-reine Verbindung der Formel I, in der R¹: CH₃; R²-CH₂CH₂-; n = Null; Z: S und R³: C₆H₅ bedeuten.

| C₇₀H₁₃N₃S (927,96) | | | | | |
|---|---|---|---|---|---|
| berechnet: | C 90,60 | H 1,41 | N 4,53 | S 3,46 | % |
| gefunden: | C 88,1 | H 1,4 | N 4,3 | S 3,4 | % |

Das Massenspektrum (FAB) zeigt eine Molmasse M = 927 (intensiver M^{⊖}-Peak bei m/z 927)
IR-Spektrum (Pulver auf KBr): *v*_{C=S}1424; *v*_{C-N}1300 cm⁻¹
Dünnschichtchromatographie: Fließmittel: Toluol/Methanol 100/3: R_{F} 0,59-0,60

### Beispiel 5

Analog wie in Beispiel 3 beschrieben wurde eine Suspension von 200 mg (0,252 mmol) der gleichen Verbindung der Formel III wie in Beispiel 3 und 1,0 g (5,34 mmol) 3-Trifluormethylphenyl-isocyanat in 205 ml Toluol 3,6 Stunden bei 70°C gerührt und anschließend aufgearbeitet und an Kieselgel 60 (H 28; Ø 2,0 cm) chromatographiert. Nach Elution mit 200 ml Toluol, 200 ml CH₂Cl₂ und 400 ml CH₂Cl₂/CH₃OH (100/1) wurden bei Elution mit 300 ml CH₂Cl₂/CH₃OH (100/1) nach dem Eindampfen 219 mg Eluatrückstand erhalten, aus dem nach analoger Bearbeitung wie in Beispiel 3 beschrieben 202 mg (81,8 % Ausbeute) DC-reine Verbindung der Formel I, in der R¹: CH₃; R²: -CH₂CH₂-; n = Null; Z: O und
R³: 3-Trifluormethylphenyl bedeuten, erhalten wurden.

| C₇₁H₁₂F₃N₃O (979,90) | | | | | |
|---|---|---|---|---|---|
| berechnet: | C 87,03 | H 1,23 | F 5,82 | N 4,29 | % |
| gefunden: | C 85,4 | H 1,6 | F 4,9 | N 3,8 | % |

Laut Massenspektrum (FAB) Molmasse M = 979 (intensiver M^{⊖}-Peak bei m/z 979)
IR-Spektrum: *v*_{C=O} 1707cm⁻¹
DC(Toluol/Methanol 10/1): R_{F} 0,52-0,54

### Beispiel 6

In analoger Weise wie in Beispiel 3 bzw. Beispiel 5 beschrieben, wurden ausgehend von 323 mg (0,4 mmol) Verbindung der Formel III (R¹:C₂H₅; R²:-CH₂CH₂-) und 1,37 g (8,4 mmol) 3,4-Methylendioxy-phenyl-isocyanat und 260 ml Toluol nach 4,7 Stunden Rühren bei 72°C, Aufarbeitung und Chromatographie an Kieselgel 60 (H 45; Ø 2,0 cm) (Elution: 500 ml Toluol, 500 ml CH₂Cl₂, je 250 ml CH₂Cl₂/CH₃OH (100:0,2); (100:0,5); (100:0,75) und (100:1); danach 500 ml CH₂Cl₂/CH₃OH (100:1), 255 mg (65,7 % Ausbeute) DC reine Verbindung der Formel I, in der R¹: C₂H₅; R²: -CH₂CH₂-; n = Null, Z: O und R³: 3,4-Methylendioxy-phenyl 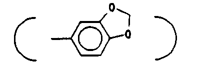
bedeuten, erhalten.

| C₇₂H₁₅N₃O₃ (969,94) | | | | |
|---|---|---|---|---|
| berechnet: | C 89,16 | H 1,56 | N 4,33 | % |
| gefunden: | C 86,3 | H 2,0 | N 4,5 | % |

Laut Massenspektrum (FAB) Molmasse M = 969 (intensiver M^{⊖}-Peak bei m/z 969)
IR-Spektrum: *v*_{C=O}1705 cm⁻¹
DC (Toluol/Methanol: 100/3) R_{F} 0,29-0,30

### Beispiel 7

Analog wie in Beispiel 3 beschrieben wurde eine Suspension von 150 mg (0,19 mmol) der gleichen Verbindung der Formel III wie in Beispiel 3 und 745 mg (3,42 mmol) 4-Chlorsulfonyl-phenyl-isocyanat in 158 ml Toluol 6,25 Stunden bei 70°C gerührt. Dann wurde die Reaktionsmischung abgesaugt, der Filterrückstand mit Toluol und Ether gewaschen und das Filtrat im Vakuum eingeengt. Der Filtratrückstand wurde in 20 ml CS₂ suspendiert, der Feststoff abgesaugt und mit CS₂ und Ether ausgewaschen und das Filtrat erneut im Vakuum eingeengt. Der danach verbliebene Rückstand wurde in 50 ml Ether suspendiert und abgesaugt, wobei 108 mg Rohprodukt der Formel I anfielen. Dieses wurde, gelöst in 20 ml Toluol, an Kieselgel 60 (H 38; Ø 2,4 cm) bei 0,35 bar N₂-Überdruck chromatographiert. Nach Elution mit je 200 ml Toluol; Toluol/Methanol (100:0,3); (100:0,6) und (100:1) wurden mit 800 ml Toluol/Methanol (100:1) nach dem Einengen 107 mg Eluatrückstand erhalten, der gemäß Beispiel 3 weiterbehandelt wurde. Man erhielt 94 mg (49,0 % Ausbeute) DC-reine Verbindung der Formel I, in der R¹: CH₃; R²: -CH₂CH₂-; n = Null; Z: O und R³: 4-Hydroxysulfonyl-phenyl 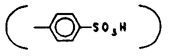
bedeuten.

| C₇₀H₁₃N₃O₄S (991,96) | | | | | |
|---|---|---|---|---|---|
| berechnet: | C 84,76 | H 1,32 | N 4,24 | S 3,23 | % |
| gefunden: | C 81,1 | H 1,6 | N 4,2 | S 3,5 | % |

Laut Massenspektrum (FAB) Molmasse M = 991 (intensiver M^{⊖}-Peak bei m/z 990)
IR-Spektrum (Pulver auf KBr) *v*_{C=O}1720; *v*_{NH,C-N}1412; *v*_{S=O}1178 cm⁻¹
DC (Toluol/Methanol 100/3) R_{F} 0,23-0,25

### Beispiel 8

Eine Suspension von 532 mg (0,67 mmol) einer Verbindung der Formel III, in der R¹CH₃ und R² (CH₂)₂ bedeutet in 400 ml Toluol wurde bei 68°C mit einer Lösung von 1,64 g (12,7 mmol) 2-Isocyanato-essigsäureethylester in 8 ml Toluol versetzt und 2 Tage bei 68-71°C und 4,9 Tage bei RT gerührt. Die schwach trübe Reaktionslösung wurde über Clarcel filtriert, auf ca. 100 ml Volumen im Vakuum eingeengt und dann auf eine Kieselgel 60/Toluol-Säule (H 42; Ø 2,0 cm) aufgezogen. Nach Elution (0,35 bar N₂-Überdruck) mit 500 ml Toluol wurden mit je 1l Toluol/Methanol (100:0,5), (100:1) und (100:2) nach dem Einengen im Vakuum 665 mg Eluatrückstand erhalten. Dieser wurde in Toluol gelöst, die Lösung über Clarcel filtriert, im Vakuum eingeengt, der verbleibende Rückstand in Ether suspendiert, und abgesaugt und mit Ether nachgewaschen. Nach Trocknen (80°, 4 Stunden, 5-7 mbar) erhielt man 588 mg (95,2 % Ausbeute) DC reine Verbindung der Formel I, in der R¹: CH₃; R²: (CH₂)₂; n = 1, Z: O; X: CH₂ und R³: COOC₂H₅ bedeuten.

| C₆₈H₁₅N₃O₃ (921,89) | | | | |
|---|---|---|---|---|
| berechnet: | C 88,59 | H 1,64 | N 4,56 | % |
| gefunden: | C 87,8 | H 2,1 | N 4,3 | % |

Massenspektrum (FAB): Molmasse M = 921 (intensiver M^{⊖}-Peak bei m/z 921)
IR-Spektrum *v*_{C=O}1712; 1745; *v*_{C=O}: 1200; *v*_{NH,C-N}1432 cm⁻¹
DC (Toluol/Methanol 100:1) R_{F} 0,09-0,13

### Beispiel 9

Eine Lösung von 540 mg (0,68 mmol) einer Verbindung der Formel III, in der R¹CH₃ und R² (CH₂)₂ bedeutet, in 1300 ml Toluol wurde bei 65°C mit einer Lösung von 2,04 g (15,8 mmol) 3-Isocyanatopropionsäuremethylester in 10 ml Toluol versetzt und 2,25 Tage bei 65°C und 2,7 Tage bei RT gerührt. Danach engte man im Vakuum die Reaktionslösung auf ca. 160 ml Volumen ein, ließ diese Lösung auf eine Kieselgel 60/Toluol-Säule (H 50; Ø 2,5 cm) aufziehen und eluierte bei 0,35 bar N₂-Überdruck nacheinander mit je 500 ml Toluol, CH₂Cl₂ und CH₂Cl₂/CH₃OH (100:0,5). Anschließend wurde mit 500 ml CH₂Cl₂/CH₃OH (100:1) eine dunkle Zone eluiert, wobei nach dem Einengen 500 mg Eluatrückstand verblieben. Dieser wurde, in Toluol gelöst, über Clarcel filtriert. Das Filtrat wurde im Vakuum eingeengt, der Rückstand in n-Heptan suspendiert und abgesaugt und 6 Stunden bei 50°C, 4-6 mbar getrocknet. Man erhielt so 456 mg (= 72,7 % Ausbeute) DC reine Verbindung der Formel I, in der R¹: CH₃; R²: (CH₂)₂; n = 1, Z: O; X: (CH₂)₂ und R³: COOCH₃ bedeuten.

| C₆₈H₁₅N₃O₃ (921,89) | | | | |
|---|---|---|---|---|
| berechnet: | C 88,59 | H 1,64 | N 4,56 | % |
| gefunden: | C 86,3 | H 2,0 | N 4,2 | % |

Massenspektrum (FAB): Molmasse M = 921 (intensiver M^{⊖}-Peak bei m/z 921 bzw. MH^{⊕-}Peak bei m/z 922)
IR-Spektrum *v*_{N-H}3350; *v*_{C=O}1704; 1733; *v*_{C=O}1200; *v*_{NH,C-N}1445 cm⁻¹
DC (CH₂Cl₂/CH₃OH: 100/4) R_{F}: 0,30-0,32

### Beispiel 10

Analog Beispiel 8 wurde ein Suspension von 586 mg (0,74 mmol) einer Verbindung der Formel III, (R¹ CH₃; R² (CH₂)₂) und 1,81 g (12,64 mmol) 3-Isocyanatopropionsäureethylester in 508 ml Toluol 2,6 Tage bei 70°C und 2,2 Tage bei RT gerührt. Anschließend wurde gemäß Beispiel 9 aufgearbeitet und chromatographiert (Säule H 53; Ø 2,5 cm). Nach Elution mit je 500 ml Toluol; CH₂Cl₂ und CH₂Cl₂/CH₃OH (100:0,3) wurden bei weiterer Elution mit je 500 ml CH₂Cl₂/CH₃OH (100:0,5) und (100:1) nach dem Einengen 535 mg Eluatrückstand erhalten, der gemäß Beispiel 9 behandelt wurde, wonach man 485 mg (70 % Ausbeute) DC reine Verbindung der Formel I erhielt, in der R¹: CH₃; R²: (CH₂)₂; n = 1; Z: O; X: (CH₂)₂ und R³: COOC₂H₅ bedeuten.

| C₆₉H₁₇N₃O₃ (935,92) | | | | |
|---|---|---|---|---|
| berechnet: | C 88,55 | H 1,83 | N 4,49 | % |
| gefunden: | C 86,6 | H 2,2 | N 4,0 | % |

Massenspektrum (FAB): Molmasse M = 935 (intensiver M^{⊖}-Peak bei m/z 935).
DC (Toluol/Methanol 100/3) R_{F}: 0,27-0,28

### Beispiel 11

Analog Beispiel 8 wurde ein Suspension von 300 mg (0,37 mmol) einer Verbindung der Formel III (R¹C₂H₅; R² (CH₂)₂) und 978 mg (7,57 mmol) 2-Isocyanatoessigsäureethylester in 245 ml Toluol 6,9 Tage bei 70-72°C und 7,9 Tage bei RT gerührt. Die Reaktionslösung wurde anschließend auf eine Kieselgel 60/Toluol-Säule (H 42; Ø 2,5 cm) aufgezogen. Nach Elution bei 0,35 bar N₂-Überdruck mit je 500 ml CH₂Cl₂ und CH₂Cl₂/CH₃OH (100:0,2) und 1l CH₂Cl₂/CH₃OH (100:0,5) wurden mit 1000 ml (100:0,5) und 200 ml (100:0,8) nach dem Eindampfen 310 mg Eluatrückstand erhalten, der gemäß Beispiel 9 behandelt wurde, wonach man 291 mg (84 % Ausbeute) DC reine Verbindung der Formel I erhielt, in der R¹: C₂H₅; R²: (CH₂)₂; n = 1; Z: O; X: CH₂ und R³: COOC₂H₅ bedeuten.

| C₆₉H₁₇N₃O₃ (935,92) | | | | |
|---|---|---|---|---|
| berechnet: | C 88,55 | H 1,83 | N 4,49 | % |
| gefunden: | C 85,8 | H 2,0 | N 4,5 | % |

Massenspektrum (FAB): Molmasse M = 935 (intensiver M^{⊖}-Peak bei m/z 935).
IR-Spektrum: *v*_{C=O}1712; 1748; *v*_{C=O}1205; *v*_{NH,C-N}1435 cm⁻¹
DC (Toluol/Methanol 100/3) R_{F}: 0,38

### Beispiel 12

Eine Lösung von 363 mg (0,45 mmol) einer Verbindung der Formel III (R¹C₂H₅; R² (CH₂)₂) und 1,28 g (9,91 mmol) 3-Isocyanatopropionsäuremethylester in 858 ml Toluol wurde 3,2 Tage bei 65°C und 2,7 Tage bei RT gerührt und danach im Vakuum auf ca. 200 ml Volumen eingeengt. Diese Lösung wurde auf eine Kieselgel 60/Toluol-Säule (H 45; Ø 2,5 cm) aufgezogen. Die Elution erfolgte bei 0,35 bar N₂-Überdruck nacheinander mit je 500 ml Toluol und CH₂Cl₂. Danach wurden nach Elution mit je 500 ml CH₂Cl₂/CH₃OH (100:0,5) und (100:1) nach dem Einengen 305 mg Eluatrückstand erhalten. Dieser wurde, gelöst in Toluol, über Clarcel filtriert und das Filtrat anschließend eingeengt. Den anfallenden Rückstand suspendierte man in Ether, saugte dann ab und wusch mit Ether nach. Nach Trocknen (8 Stunden, 60°C, 4-6 mbar) erhielt man 288 mg (= 68,4 % Ausbeute) DC reine Verbindung der Formel I, in der R¹: C₂H₅; R²: (CH₂)₂; n = 1; Z: O; X: (CH₂)₂ und R³: COOCH₃ bedeuten.

| C₆₉H₁₇N₃O₃ (935,92) | | | | |
|---|---|---|---|---|
| berechnet: | C 88,55 | H 1,83 | N 4,49 | % |
| gefunden: | C 88,4 | H 2,3 | N 4,4 | % |

Massenspektrum (FAB): Molmasse M = 935 (intensiver M^{⊖}-Peak bei m/z 935).
DC (CH₂Cl₂/CH₃OH: 100/3) R_{F}: 0,39-0,42

### Beispiel 13

Eine Suspension von 182 mg (0,23 mmol) einer Verbindung der Formel III (R¹ CH₃; R² (CH₂)₂) und 935 mg (4,6 mmol) 2-Isocyanato-4-methylthiobuttersäureethylester in 188 ml Toluol wurde 6,17 Stunden bei 71°C gerührt. Anschließend wurde die Lösung im Vakuum eingeengt, der verbliebene Rückstand in Ether suspendiert, abgesaugt und mit Ether ausgewaschen. Der Feststoff (203 mg) wurde in 50 ml Toluol gelöst auf eine Kieselgel 60/Toluol-Säule (H 44; Ø 2,5 cm) aufgezogen. Die Elution erfolgte bei 0,35 bar N₂-Überdruck nacheinander mit 200 ml Toluol und je 500 ml Toluol/Methanol (100:0,2), (100:0,3) und (100:0,4). Bei weiterer Elution mit 1l Toluol/Methanol (100:0,4) wurden nach dem Eindampfen 63 mg Eluatrückstand erhalten, der in Ether suspendiert, abgesaugt und 8 Stunden bei 80°C, 4-7 mbar getrocknet wurde. Man erhielt so 43 mg (18,8 % Ausbeute) DC reine Verbindung der Formel I, in der R¹: CH₃; R²: (CH₂)₂; n = 1; Z: O; X: 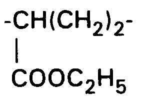
und
R³: SCH₃ bedeuten.

| C₇₁H₂₁N₃O₃S (996,04) | | | | | |
|---|---|---|---|---|---|
| berechnet: | C 85,62 | H 2,13 | N 4,22 | S 3,22 | % |
| gefunden: | C 84,0 | H 2,1 | N 4,3 | S 3,8 | % |

Massenspektrum (FAB): Molmasse M = 995 (intensiver M^{⊖}-Peak bei m/z 995)
DC (Toluol/Methanol 100/3) R_{F}: 0,32-0,34

### Beispiel 14

Eine Suspension von 190 mg (0,24 mmol) einer Verbindung der Formel III (R¹ CH₃; R²(CH₂)₂) und 1,0 g (7,49 mmol) Chlor-tert.-butyl-isocyanat in 185 ml Toluol wurde 1,93 Tage bei 70-71°C gerührt und anschließend bei RT filtriert. Das Filtrat wurde im Vakuum eingeengt, der verbliebene Rückstand in Ether suspendiert und der Feststoff abgesaugt. Dieser (224 mg) wurde, gelöst in 130 ml Toluol, auf eine Kieselgel 60/Toluol-Säule (H 43; Ø 2,5 cm) aufgezogen. Die Elution erfolgte bei 0,35 bar N₂-Überdruck nacheinander mit 500 ml Toluol, 500 ml Toluol/Methanol (100:0,1) und 800 ml (100:0,2). Bei weiterer Elution mit 1l Toluol/Methanol (100:0,2) wurde nach dem Einengen 155 mg Eluatrückstand erhalten, der in Ether suspendiert, abgesaugt und 8 Stunden bei 80°C, 6-7 mbar getrocknet wurde. Man erhielt so 140 mg (63 % Ausbeute) DC reine Verbindung der Formel I, in der R¹: CH₃; R²: (CH₂)₂; n=1; Z: O; X: -C(CH₃)₂-CH₂- und R³: Cl bedeuten.

| C₆₈H₁₆ClN₃O (926,34) | | | | |
|---|---|---|---|---|
| berechnet: | C 88,17 | H 1,74 | Cl 3,83 | N 4,54 |
| gefunden: | C 87,1 | H 1,5 | Cl 4,3 | N 4,3 |

Massenspektrum (FAB): Molmasse M = 925; 927 (intensiver M^{⊖}-Peak bei m/z 925) (bezogen auf ³⁵Cl).
IR-Spektrum: *v*_{C=O}1696; *v*_{NH,C-N}1422 cm⁻¹,
DC (Toluol/Methanol 100/3) R_{F} 0,46-0,47

### Beispiel 15

Eine Suspension von 190 mg (0,235 mmol) einer Verbindung der Formel III (R¹C₂H₅; R² (CH₂)₂) und 1,195 g (7,286 mmol) 2-Bromisopropyl-isocyanat in 155 ml Toluol wurde 1,9 Tage bei 70°C gerührt und anschließend im Vakuum eingeengt. Den Rückstand suspendierte man in Ether; der Feststoff wurde abgesaugt, mit Ether ausgewaschen und in 150 ml Toluol gelöst auf eine Kieselgel 60/Toluol-Säule (H 43; Ø 2,5 cm) aufgezogen. Die Elution erfolgte bei 0,35 bar N₂-Überdruck nacheinander mit je 500 ml Toluol und Toluol/Methanol (100:0,1). Bei weiterer Elution mit 600 ml Toluol/Methanol (100:0,1) wurden nach dem Eindampfen 65 mg Eluatrückstand erhalten, der in Ether suspendiert, abgesaugt und 8 Stunden bei 80°C, 6-8 mbar getrocknet wurde. Man erhielt so 54 mg (23,7 % Ausbeute) DC reine Verbindung der Formel I, in der R¹: C₂H₅; R²: (CH₂)₂; n = 1; Z: O; X: -CH(CH₃)-CH₂- und R³: Br bedeuten.

| C₆₈H₁₆BrN₃O (970,8) | | | | | |
|---|---|---|---|---|---|
| berechnet: | C 84,13 | H 1,66 | Br 8,23 | N 4,33 | % |
| gefunden: | C 79,3 | H 1,7 | Br 10,1 | N 4,5 | % |

Massenspektrum (FAB): Molmasse M = 969; 970; 971; 972 (intensive M^{⊖}-Peaks bei m/z 969; 970; 971 und 972) (bedingt durch die Isotope des Broms)
DC (Toluol/Methanol 100/3) R_{F} 0,34-0,36

## Patentansprüche

1. Verbindung der Formel I und/oder II 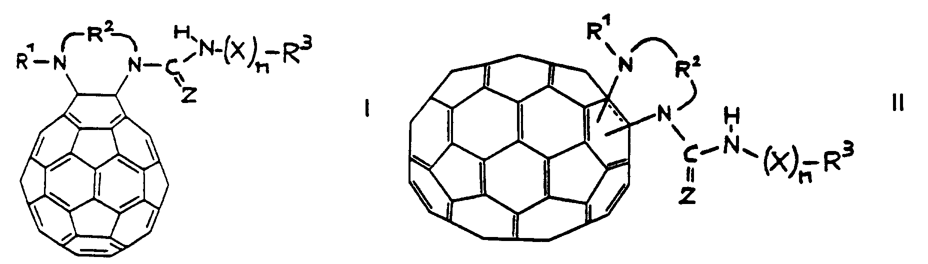 in denen
R¹ (C₁-C₄)-Alkyl ist,
R² (C₂-C₄)-Alkylen oder 1,2-Cyclo-(C₃-C₇)-alkylen ist,
n eine ganze Zahl Null oder 1 ist,
Z O oder S ist,
X (C₁-C₁₂)-Alkylen, (C₃-C₁₂)-Alkenylen oder (C₆-C₁₀)-Arylen ist, wobei Alkylen und Alkenylen gegebenenfalls durch (C₁-C₄)-Alkoxy, (C₁-C₄)-Alkylthio, Cl, Br, J, (C₁-C₄)-Alkoxycarbonyl, (C₆-C₁₀)-Aryl und/oder (C₆-C₁₀)-Aryl-(C₁-C₄)-alkyl substituiert ist,
wobei Aryl gegebenenfalls ein- oder mehrfach unabhängig voneinander substituiert ist durch Halogen, (C₁-C₄)-Alkyl, (C₁-C₄)-Alkoxy, CF₃, CN oder NO₂,
und wobei Arylen gegebenenfalls ein- oder mehrfach unabhängig voneinander substituiert ist durch Halogen, (C₁-C₄)-Alkyl, (C₁-C₄)-Alkoxy, (C₁-C₄)-Alkylthio, CF₃, OCF₃, CN, NO₂ und/oder (C₁-C₄)-Alkoxycarbonyl,
R³ falls n Null ist,
(C₁-C₁₂)-Alkyl, (C₃-C₁₁)-Alkenyl, (C₃-C₈)-Alkinyl, (C₁-C₈)-Perfluoralkyl, (C₆-C₁₄)-Aryl oder Trimethylsilyl ist, wobei Alkyl, Alkenyl und Alkinyl gegebenenfalls ein- oder mehrfach unabhängig voneinander durch (C₁-C₄)-Alkoxy, (C₁-C₄)-Alkylthio, Phenyloxy, Phenylthio, Halogen, ClSO₂ und/oder (C₁-C₄)-Alkoxycarbonyl, und Aryl gegebenenfalls ein- oder mehrfach unabhängig voneinander durch Halogen,(C₁-C₄)-Alkyl, welches gegebenenfalls durch Halogen substituiert ist, (C₁-C₄)-Alkoxy, NO₂, CN, OCF₃, ClSO₂, SO₃H, (C₁-C₄)-Alkanoyloxy, (C₁-C₄)-Alkoxycarbonyl und/oder
(C₁-C₂)-Alkylendioxy substituiert ist,
R³ falls n = 1 ist,
Wasserstoff, CH₃, welches gegebenenfalls durch Halogen substituiert ist, CH = CH₂, (C₁-C₄)-Alkoxy, (C₁-C₄)-Alkylthio, Halogen, (C₆-C₁₄)-Aryl, (C₆-C₁₄)-Aryloxy, (C₆-C₁₄)-Aryl-(C₁-C₄)-alkyloxy, Trimethylsilyloxy, C(O)-NHR', C(O)NR'₂, COOR', worin R' (C₁-C₄)-Alkyl bedeutet, (C₁-C₆)-Alkanoyloxy, SO₂R⁵, OSO₂R⁵, worin R⁵ (C₁-C₄)-Alkyl, (C₆-C₁₀)-Aryl oder (C₁-C₄)-Alkyl-(C₆-C₁₀)-aryl bedeutet, oder -N = C = Z, worin Z die obengenannte Bedeutung hat, und Aryl gegebenenfalls ein- oder mehrfach unabhängig voneinander durch Halogen, (C₁-C₄)-Alkoxy, (C₁-C₄)-Alkylthio, (C₁-C₄)-Alkyl, welches gegebenenfalls durch Halogen substituiert ist, NO₂, CN, ClSO₂, SO₃H, (C₁-C₄)-Alkanoyloxy, (C₁-C₄)-Alkoxycarbonyl oder (C₆-C₁₀)-Aryloxy substituiert ist.

2. Verbindung der Formel I und/oder II gemäß Anspruch 1, dadurch gekennzeichnet, daß
Z 0 ist,
X (C₁-C₈)-Alkylen, gegebenenfalls durch (C₁-C₄)-Alkoxy, (C₁-C₄)-Alkylthio, (C₁-C₄)-Alkoxycarbonyl oder Cl substituiert, oder o-, m- oder p-Phenylen ist, gegebenenfalls substituiert durch Halogen, (C₁-C₄)-Alkoxy, (C₁-C₄)-Alkyl oder CF₃, und
R³ falls n Null ist,
(C₁-C₁₂)-Alkyl, Allyl, Phenyl, Benzyl oder Naphthyl ist, wobei Alkyl gegebenenfalls unabhängig voneinander einfach oder zweifach durch (C₁-C₄)-Alkoxy, (C₁-C₄)-Alkylthio, Phenyloxy, Phenylthio, Cl, Br oder (C₁-C₄)-Alkyloxycarbonyl substituiert ist, und Phenyl, Benzyl oder Naphthyl gegebenenfalls ein- oder mehrfach unabhängig voneinander durch Halogen, (C₁-C₄)-Alkoxy, (C₁-C₄)-Alkyl, CF₃, NO₂, CN, (C₁-C₄)-Alkanoyloxy, ClSO₂, SO₃H und/oder (C₁-C₄)-Alkoxycarbonyl oder einfach durch Methylendioxy substituiert ist, und
R³ falls n = 1 ist,
Wasserstoff, CH₃, ClCH₂, BrCH₂, Vinyl, (C₁-C₄)-Alkoxy, (C₁-C₄)-Alkylthio, Cl, Br, Phenoxy, Benzyloxy, Phenyl, Naphthyl, C(O)NHR', C(O)NR'₂ oder COOR', worin R' (C₁-C₄)-Alkyl bedeutet, ist und wobei Phenyl oder Naphthyl gegebenenfalls ein- oder mehrfach unabhängig voneinander durch (C₁-C₄)-Alkoxy, (C₁-C₄)-Alkyl, CF₃, NO₂, CN, ClSO₂, SO₃H und/oder (C₁-C₄)-Alkoxycarbonyl substituiert ist, und R¹, R² und n die obengenannte Bedeutung haben.

3. Verbindung der Formel I und/oder II gemäß Anspruch 1 oder 2, dadurch gekennzeichnet, daß
R¹ CH₃ oder C₂H₅ ist,
R² (C₂-C₃)-Alkylen oder 1 ,2-Cyclo-(C₅-C₆)-alkylen ist,
n eine ganze Zahl Null oder 1 ist,
Z O ist,
X (C₁-C₇)-Alkylen, gegebenenfalls durch CH₃O, CH₃S, COOCH₃ oder COOC₂H₅ substituiert, oder o-, m- oder p-Phenylen ist,
R³ falls n Null ist,
(C₁-C₈)-Alkyl, Allyl, Phenyl, Benzyl oder Naphthyl ist, wobei Alkyl gegebenenfalls einfach oder zweifach unabhängig voneinander durch CH₃O, CH₃S, COOCH₃ oder COOC₂H₅ substituiert ist, und Phenyl, Benzyl oder Naphthyl gegebenenfalls einfach oder zweifach unabhängig voneinander durch F, Cl, Br, CH₃O, C₂H₅O, CH₃, C₂H₅, NO₂, ClSO₂, SO₃H, CF₃, COOCH₃ und/oder COOC₂H₅ oder 1,2-O-CH₂-O substituiert ist, und
R³ falls n = 1 ist,
Wasserstoff, CH₃, CH₃O, CH₃S, Cl, Br, C₆H₅O, C₆H₅CN₂O, Phenyl, COOCH₃, COOC₂H₅, C(O)NHR', C(O)N-R'₂, wobei R' (C₁-C₄)-Alkyl bedeutet, ist, und Phenyl gegebenenfalls durch CH₃O, C₂H₅O, CH₃, CF₃, NO₂, ClSO₂, COOCH₃ oder COOC₂H₅ substituiert ist.

4. Verbindung der Formel I gemäß einem oder mehreren der Ansprüche 1, 2 oder 3.

5. Verfahren zur Herstellung einer Verbindung der Formel I und/oder II gemäß Anspruch 1, dadurch gekennzeichnet, daß man eine Verbindung der Formel III und/oder IV 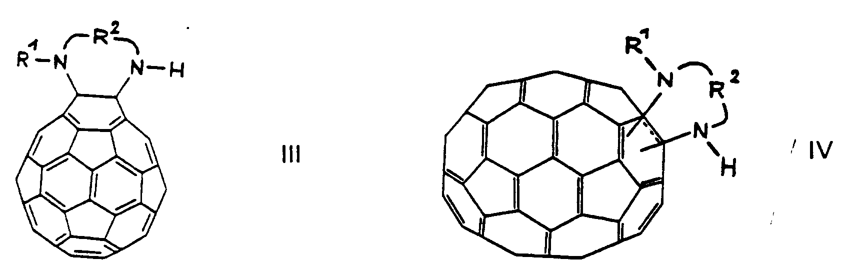 in denen
R¹ und R² die obengenannte Bedeutung haben, mit einem Isocyanat oder Isothiocyanat der Formel V
Z = C = N - (X)ₙ - R³ V
in der Z, n, X und R³ die obengenannte Bedeutung haben, zu einer Verbindung der Formel I und/oder II umsetzt.

6. Verfahren gemäß Anspruch 5, dadurch gekennzeichnet, daß die Umsetzung in einem inerten Löse- oder Suspensionsmittel durchgeführt wird.

7. Verfahren gemäß Anspruch 5 oder 6, dadurch gekennzeichnet, daß die Umsetzung bei einer Temperatur von -30°C bis 300°C durchgeführt wird.

8. Verfahren gemäß einem oder mehreren der Ansprüche 5, 6 oder 7, dadurch gekennzeichnet, daß das Molverhältnis zwischen den Verbindungen der Formel III und/oder IV und den Verbindungen der Formel V 1:1 bis 1:1000 beträgt.

9. Verwendung der Verbindung gemäß Anspruch 1 zur Modifizierung von Katalysatoren.

10. Verwendung der Verbindung gemäß Anspruch 1 zur Hemmung von Enzymen.
